(19) Europäisches Patentamt

European Patent Office

Office européen des brevets

(11) Publication number: **0 320 685 B1**

## EUROPEAN PATENT SPECIFICATION

(45) Date of publication of patent specification: **13.10.93**   (51) Int. Cl.5: **C12N 9/28**

(21) Application number: **88119794.1**

(22) Date of filing: **28.11.88**

The file contains technical information submitted after the application was filed and not included in this specification

(54) **A process for the obtention of thermostable alpha-amylases by culturing superproductive microorganisms at high temperatures.**

(30) Priority: **07.12.87 ES 8703510**

(43) Date of publication of application:
**21.06.89 Bulletin 89/25**

(45) Publication of the grant of the patent:
**13.10.93 Bulletin 93/41**

(84) Designated Contracting States:
**BE DE FR GB IT NL**

(56) References cited:
**EP-A- 0 140 610**
**EP-A- 0 169 920**

(73) Proprietor: **COMPANIA ESPANOLA DE PETROLEOS S.A. -CEPSA**
**Clara del Rey, 31**
**E-28002 Madrid(ES)**

(72) Inventor: **Perez Pascual, Miguel Antonio**
**C/ de los Curas, 58-D**
**Torrejon De Ardoz Madrid(ES)**
Inventor: **Del Castillo Roman, Francisco**
**C/ San Félix de Alcalá, 1**
**Alcala de Henares Madrid(ES)**
Inventor: **Martinez Reif, Pilar**
**Avda. de Bruselas, 73**
**Madrid(ES)**
Inventor: **Murillo Aranjo, Francisco José**
**Avda. Monte Carmelo**
**Edificio Estrella Polar**
**Murcia(ES)**
Inventor: **Ruiz Vazquez, Rosa Maria**
**Avda. Monte Carmelo**
**Edificio Estrella Polar**
**Murcia(ES)**
Inventor: **Balsalobre Balibrea, Josefa Maria**
**c/ Eras, 60**
**Edificio Enri**
**Alcantarilla Murcia(ES)**

(74) Representative: **Ungria Lopez, Javier et al**
**Avda. Ramon y Cajal, 78**
**E-28043 Madrid (ES)**

**Description**

1. OBJECT OF THE INVENTION.

The object of this invention is an industrial process for obtaining an enzyme α-amylase by means of thermophilic microorganisms that super-produce the enzyme at temperatures ranging between 50 and 70°C, especially when the culture media contain corn steep liquor and ammonium chloride.

2. TECHNICAL-ECONOMIC JUSTIFICATION.

The use of amylases in starch degradation processes is a classic process that has been used with a great number of purposes, such as the production of oligosaccharide, maltose and glucose-rich syrups.α-amylases in particular are enzymes of a notorious commercial interest for their use in the conversion of starch into water-soluble sugars, in particular for the conversion thereof into glucose.

The industrial process for starch hydrolysis requires the use of energetic conditions, with temperatures ranging between 105 and 115°C, in order to attain the complete gelatinization of the granules. Said operating conditions have led to the selection of different Bacillus species producing thermostable α-amylases. In British Patent No. 1,296,839 of NOVO or in Spanish Patent No. 526,406, U.S. No. 4,642,288 and European Patent Application No. 843068636 of CEPSA, different processes for the production of thermostable α-amylases from cultures of different Bacillus species have been described. The enzymes produced by these microorganisms are especially attractive for their use in starch liquefaction processes. They are much more stable than the early α-amylases obtained from different varieties of Bacillus. The production level of those strains is, however, low, and therefore it is essential to conduct a selection of superproductive mutants as well as to conceive a suitable culture medium.

Thus it has been discovered that the Bacillus sp. strain NCIB 11866, described in Spanish Patent No. 526,406, U.S. No. 4,642,288 and European Patent Application No. 843068636 of CEPSA, is capable of mutation by different mutagenic agents that allow the commercial production of the parental strain thermostable α-amylase. These mutants can be, besides, cultured in corn steep liquor and ammonium chloride-rich industrial culture media.

3. TECHNICAL DESCRIPTION

The commercial production of thermostable α-amylases of Bacillus sp. strains NCIB 11886 and 11887, as described in Spanish Patent No. 526,406, U.S. No. 4,642,288 and European Patent Application No. 843068636 of CEPSA, needs the previous obtention of superproductive mutants, as well as a specific culture medium and suitable purification and recovery conditions.

So as to obtain superproductive mutants, Bacillus sp. strains NCIB 11886 and 11887 have been submitted to different mutagenic treatments, under the most effective conditions found. The mutagenic agents most used have been N-methyl-N'-nitro-N-nitroso-guanidine (NG) and ultraviolet light (UV). All treatments have been carried out with exponential cultures. The convenience of using exponential cultures for the mutagenic treatment derives from the recognized higher effectiveness of many mutagenic agents, particularly of NG, on bacterial cultures in exponential phase. The isolation of cells surviving the treatment is made in solid culture medium in Petri dishes. With a starch-rich medium, superproductive mutants can be directly identified, since they produce amyl-lysis halos around the colonies. The size of these halos indicates the α-amylase synthesis ability of the colony.

The election of an efficient selection medium is essential for developing a mutagenesis programme. The method must be specific, simple and efficient. Numerous culture media allow the detection of α-amylase activity, provided they contain starch. But, before the necessity to observe a great number of colonies per plate and distinguish enzyme productivity differences, we have developed an specific culture medium. This medium contains glucose, apart from starch, whereby the size of amyl-lysis halos is very noticeably reduced without affecting colony growth.

Bacillus sp. strain NCIB 11886 mutagenic treatments led to the isolation of 4700 colonies according to the above mentioned conditions. Among them, Bacillus sp. strain NCIB 12563 was isolated, as it displayed an amyl-lysis halo significantly wider than the rest. Said strain is deposited at the National Collection of Industrial Bacteria, Torry Research Station, 135 Abbey Road, Aberdeen AB9 8DE, Escocia.

The α-amylase superproductive character of strain NCIB 12563 has been confirmed in numerous liquid culture media, both in Erlenmeyer and in fermenter. In general, strain NCIB 12563 productivity is 2 to 4 times higher than that of the parental strain. In all cases, the α-amylase enzyme activity was determined

2

following the protocol described in Spanish Patent No. 526,406, U.S. No. 4,642,288 and European Patent Application No. 843068636 of CEPSA:

A 4% (weight/ volume) solution of starch in 0.02 M phosphate buffer, pH 5.7, with a 0.006 M NaCl concentratin, is heated up to about 50°C. Likewise, the culture broth, the activity of which is intended to be determined (duly diluted in the same broth as the starch) is heated up to the same temperature. Afterwards, 0.5 ml of the problem enzyme suspension and 0.5 ml of the starch suspension, both previously heated, are poured onto a test tube. The mixture is incubated for 5 minutes at 50°C, with magnetic stirring. The reaction is stopped by a sharp drop of temperature and addition of 0.1 ml of Bernfeld's reagent. Said reagent was prepared as follows: a 16 g NaOH/200 ml $H_2O$ solution (in all cases throughout this patent destilled water was used) is poured onto 300 ml of water near boiling, then 10 g of 3,5-dinitrosalicylic acid and 300 g of Rochelle salt (sodium potassium tartrate) are added and the mixture is heated until complete dissolution of the reagent and, finally, it is leveled to a liter. Problem samples are then carried to a violently boiling water bath for 5 minutes. After that time has passed, the samples are cooled and 10 ml of $H_2O$ are added. Finally, the absorption of each sample is determined at 540 nm versus a standard prepared with $H_2O$ and Bernfeld's reagent.

The CEPSA α-amylase unit is defined as the amount in mg of reducing sugars, measured as maltose, yielded per minute under the above specified assay conditions. Thus,

$$CEPSA \; \alpha\text{-amylase units/ml broth} = \frac{mg \; maltose \; yielded/ml \times D \times d}{t}$$

wherein,

D : dilution made in the culture broth.

d : dilution in the reaction medium itself.

t : time of reaction (min).

Therefore, 540 nm absorption units of each sample should be converted into mg of reducing sugars, measured as maltose. With this aim, some maltose standards are prepared with concentrations ranging between 0.5 and 5.0 mg/ml and their actual content in reducing sugars is determined by the Bernfeld's reagent. A standard straight line is obtained thus that relates the absorption at 540 nm to the reducing sugar content, measured with respect to maltose. From said line the actual content in reducing sugars of each samble is determined by interpolation, once the A540 corresponding to the sugars present in the starch or in the culture broth has been discounted from the measure for each case. That is to say, that:

$$(A_{540})_{actual} = (A_{540})_{measured} - (A_{540})_{starch} - (A_{540})_{broth}$$

The strain NCIB 12563 conserves the thermophilic characteristics of the parental strain, as described in Spanish Patent No. 526,406, U.S. No. 4,642,288 and European Patent Application No. 843068636 of CEPSA, which enables it to be cultured at temperatures between 50 and 70°C and, preferably, between 56 and 62°C. The culturing time can be thus noticeably short, between 16 and 30 hours, which gives these mutants and their derivatives a high commercial interest.

Culture media used consist of a carbon source, a nitrogen source and salts. The main carbon sources used are complex polysaccharides, such as starch, or products resulting from its partial hydrolysis. For culturing NCIB 12563, maltodextrins with a D.E. (dextrose-equivalent) ranging between 5 and 20% and, preferably, between 10 and 15%, have turned out to be specially useful. The amounts used vary between 1 and 4% w/v and, preferably, between 2 and 3%. As nitrogen source different organic and inorganic nutrients have been used. Among the former, yeast extracts, soybean meal and corn steep liquor are included. Among the latter, different ammonium salts.

One of the outstanding aspects of the present invention is the discovery of the positive effect of the simultaneous addition of corn steep liquor and ammonium ions to the culture medium. The corn steep liquor is added at concentrations from 2 to 12% and, preferably, from 4 to 5%. By their side, ammonium ions are added in the form of ammonium chloride at concentrations from 1 to 10 g/l and, preferably, from 2 to 4 g/l. This ammonium ions supply can be carried out in other ways. Thus, if one wishes to pre-treat the corn steep liquor by any usually employed method, it is possible to use ammonium hydroxide in the alkaline treatment.

Thus according to the invention the strain NCIB 12563 is cultured in the suitable medium and under aerobic conditions. Stirred erlenmeyers or, usually, fermenters, are used for this purpose. In this case, the usual aeration is of 1.4 air volumes per medium volume per minute.

The enzyme produced by the strain NCIB 12563 is recovered from the culture broth by a solid-liquid separation stage. This separation can be carried out both by filtration and by centrifugation. The recovered enzyme is then concentrated either with simultaneous purification or not. If it is only intended to concentrate the enzyme, the centrifugated or filtrated broth is evaporated under vacuum. If, besides concentrating, it is desired to purify the $\alpha$-amylase, the centrifugated or filtrated broth can be fractionated with the use of organic solvents or ultracentrifugation.

The following examples illustrate the invention, without having a limitative character. It is obvious that changes and modifications known by those skilled in the art can be introduced.

Example 1

The strain NCIB 11886 has been mutated in order to obtain $\alpha$-amylase superproductive strains. Culture media having been used are expressed in Table 1.

Table 1

| Culture Media. | | | |
|---|---|---|---|
| | AM-8 | AM-79 | AM-138 |
| Soluble starch | 40.0 g/l | 5.0 | |
| D.E. 15% Maltodextrin | | | 20.0 |
| Glucose | 35.0 | | |
| Yeast extract | | 5.0 | |
| Protease-peptone | 10.0 | | |
| Corn Steep Liquor | | | 45.0 |
| Trizma base | | 17,8 | |
| $NH_4Cl$ | | | 3.0 |
| KCl. | | 0.5 | 0.5 |
| $MgCl_2.6H_2O$ | | 3.2 | 3.2 |
| $CaCl_2$ | | 1.0 | 1.0 |
| $MnSO_4$ | | 0.5 | 0.5 |
| Bacteriological Agar | 20.0 | | |
| pH | 7.5 | 7.2 | 6.8 |

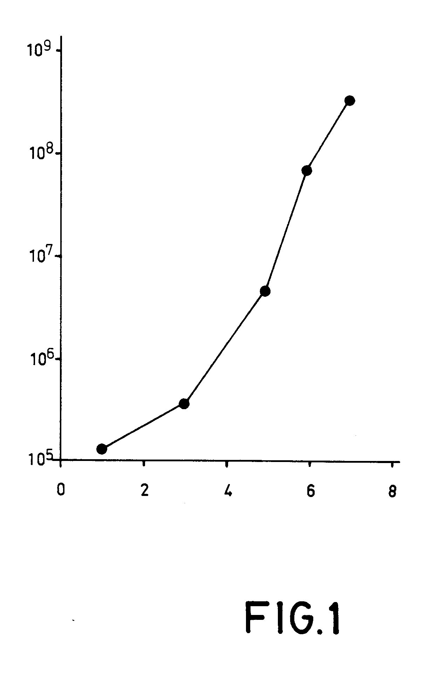

10 ml of AM-79 culture medium are inoculated, previously heated to 56°C, in a 50 ml erlenmeyer with a colony of the strain NCIB 11886 and it is incubated overnight at 56°C with orbital agitation of 230 rpm. The resulting culture in stationary phase is 100-fold diluted in 10 ml of AM-79 medium under the same conditions as above described and is cultured until the exponential growth phase is half-completed, i.e., for 6 incubation hours. Figure 1 shows the growth of strain NCIB 11886 in AM-79 culture medium. The number of viable cells per ml should be read in the ordinate axis and the time of culture in hours in the abscissa axis.

5 ml of exponential culture are centrifugated and the resulting cells are re-suspended in 5 ml of AM-79 medium preheated to 56°C.

For NG treatment, 100 ug of said mutagenic agent are added to the cell suspension and it is incubated at 56°C for 20 minutes. The strain NCIB 12563 was later isolated by seeding into AM-8 medium-containing Petri dishes, due to its high amyl-lytic activity.

Example 2

The over-production of mutant NCIB 12563 with respect to the parental strain NCIB 11886 has been checked in complex liquid culture media in erlenmeyer.

With this purpose, 10 ml of AM-79 culture medium preheated to 56°C in a 50 ml erlenmeyer are inoculated with a colony of strain NCIB 12563 and it is incubated at 56°C with an orbital agitation of 230 rpm. Aliquots are taken at different times of incubation for determining the production of $\alpha$-amylase. The results of Table 2 show that the superproductive mutant NCIB 12563 produces about four times more $\alpha$-amylas than the parental strain NCIB 11886.

4

Table 2

| α-amylase production of strains NCIB 11886 and 12563 in AM-79 liquid medium in erlenmeyer | | |
|---|---|---|
| STRAIN | Culturing time (hours) | |
| | 24 | 48 |
| NCIB 11886<br>NCIB 12563 | 216 u/ml.<br>917 u/ml. | 213 u/ml.<br>878 u/ml. |

Example 3

The overproduction of mutant NCIB 12563 with respect to the parental strain NCIB 11886 has been checked in complex liquid media in fermenters.

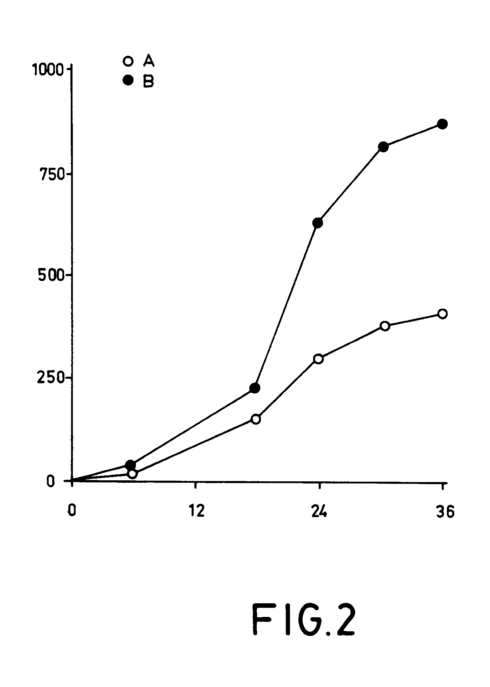

Therefor, 100 ml of AM-79 culture, preheated to 56°C, are inoculated in a 500 ml erlenmeyer with a colony of strain NCIB 12563 and it is incubated at 56°C with orbital agitation of 230 rpm. overnight. The resulting stationary culture is 25-fold diluted in AM-96 culture medium in a 2 l. fermenter (useful volume: 1125 ml). The fermenter culture is incubated at 56°C with an aeration of 1.3 v/v/min., agitation of 1750 rpm. and pH maintained above 7.2 by addition of 10% NaOH. Aliquots are taken at different times of incubation in order to determine the production of α-amylase. Figure 2 shows the production of α-amylase by strains NICB 11886 (curve A) and NCIB 12563 (curve B), the CEPSA α-amylase units per ml being represented in the ordinate axis and the time of culture in hours in the abscissa axis. Such as it can be seen, the superproductive mutant NCIB 12563 produces twice more α-amylase than the parental strain, reaching the maximum production at the same mutation time: 36 hours.

Example 4

α-amylase production by the superproductive strain NCIB 12563 becomes possible in industrial simple culture media.

A NCIB 12563 colony is therefor grown in a 500 ml. flask witn 100 ml of AM-79 medium, as described in Example 3. A second AM-79 flask is then inoculated with a 5% volume of the former culture and it is incubated for 8 hours under identical conditions. A 2 l. fermenter containing AM-138 medium is inoculated with this culture. A 10% volume is used for this inoculation. The fermenter culture is conducted under the following conditions: pH 6.8 and 58°C. The development of the solved oxygen is free, with an agitation of 1250 rpm. and an aeration of 1.4 vol/vol/min. of air. After 4 hours of culture, an aliquot thereof is taken for inoculating, with a 4% v/v, a 15 l. fermenter. The latter contains too AM-100 medium and it is cultured at 58°C and pH 6.8. The development of the solved oxygen is free, the agitation is of 650 rpm. and the aeration is of 1.4 v/v/min of air. After 20 hours of culturing, 1100 u/ml are obtained.

Example 5

The concentration and purification of NCIB 12563 -amylase can be conducted by fractionating with organic solvents. A culture broth is used therefor such as the one obtained in Example 4. Said broth is centrifugated in a disk centrifuge, such as the Westfalia equipment Mod. SAOOH-205, with a 30 l/h flow. The supernatant is precipitated with acetone at 4°C, recovering the fraction between 44 and 60 v/v of acetone. The product contains a 87% of the initial α-amylase.

Example 6

The concentration of thermostable α-amylase of NCIB 12563, can be carried out by evaporation under vacuum at high temperatures. A culture broth such as the one obtained in Example 4 is used therefor, it is centrifuged as described in Example 5 and the obtained supernatant is concentrated by evaporation under reduced pressure.

The concentration is carried out by maintaining the evaporation temperature at 60°C. The supernatant is concentrated up to 6 times. The overall yield, from the culture broth, is of 95%.

**Claims**

1.  A process for the obtainment of thermostable α-amylases by culturing the superproducing microorganism Bacillus sp. NCIB 12563 at high temperatures and in a suitable commercial medium for producing the enzyme.

2.  A process according to the previous claim characterised in that the culturing is done in a suitable medium at temperatures between 50 and 70° C and preferably between 55 and 62° C.

3.  A process according to the previous claims characterised in that the culturing of the microorganism Bacillus sp. NCIB 12563 is done in a medium which contains corn steep liquor and ammonium chloride as nitrogen sources.

4.  A process according to the previous claim characterised in that the culturing is done in a medium which contains between 2 and 12 % corn steep liquor and preferably between 4 and 5%, and between 0.1 and 1.0 % of ammonium chloride, and preferably between 0.2 and 0.4 %.

5.  A process according to the previous claims, characterised in that the α-amylase produced is recovered, concentrated and purified from the culture medium.

**Patentansprüche**

1.  Verfahren zum Erhalten von thermostabilen α-Amylasen durch Kultivieren des überproduzierenden Mikroorganismus Bacillus sp. NCIB 12563 bei hohen Temperaturen und in einem geeigneten kommerziellen Medium zum Produzieren des Enzyms.

2.  Verfahren nach dem vorhergehenden Anspruch, **dadurch gekennzeichnet,** daß des Kultivieren in einem geeigneten Medium bei Temperaturen zwischen 50 und 70°C und vorzugsweise zwischen 55 und 62°C durchgeführt wird.

3.  Verfahren nach den vorhergehenden Ansprüchen, **dadurch gekennzeichnet,** daß des Kultivieren des Mikroorganismus Bacillus sp. NCIB 12563 in einem Medium durchgeführt wird, welches Maisquellwasser und Ammoniumchlorid als Stickstoffquellen enthält.

4.  Verfahren nach dem vorhergehenden Anspruch, **dadurch gekennzeichnet,** daß das Kultivieren in einem Medium durchgeführt wird, welches zwischen 2 und 12 % und vorzugsweise zwischen 4 und 5 % Maisquellwasser und zwischen 0,1 und 1,0 % und vorzugsweise zwischen 0,2 und 0,4 % Ammoniumchlorid enthält.

5.  Verfahren nach den vorhergehenden Ansprüchen, dadurch gekennzeichnet, daß die produzierte α-Amylase aus dem Kulturmedium gewonnen, konzentriert und gereinigt wird.

**Revendications**

1.  Procédé d'obtention d'α-amylases thermostables par culture du micro-organisme surproducteur Bacillus sp. NCIB 12563 à des températures élevées et dans un milieu commercial approprié pour produire l'enzyme.

2.  Procédé selon la revendication précédente, caractérisé en ce que la culture est opérée dans un milieu approprié à des températures comprises entre 50 et 70°C et de préférence entre 55 et 62°C.

3.  Procédé selon les revendications précédentes, caractérisé en ce que la culture du micro-organisme Bacillus sp. NCIB 12563 est opérée dans un milieu contenant du liquide de macération de maïs et du chlorure d'ammonium comme sources d'azote.

4.  Procédé selon la revendication précédente, caractérisé en ce que la culture est opérée dans un milieu qui contient entre 2 a 12 % de liquide de macération de maïs et de préférence entre 4 et 5 %, et entre 0,1 et 1,0 % de chlorure d'ammonium, et de préférence entre 0,2 et 0,4 %.

5. Procédé selon les revendications précédentes, caractérisé en ce que l'$\alpha$-amylase produite est récupérée, concentrée et purifiée dans le milieu de culture.

FIG.1

FIG.2